# EUROPEAN PATENT SPECIFICATION

(11) **EP 3 468 656 B1**
(45) Date of publication and mention of the grant of the patent: **07.04.2021**
(21) Application number: 17719555.9
(22) Date of filing: 21.04.2017
(51) Int. Cl.: A61B 17/34, A61M 5/32, A61M 25/06, A61M 39/10

(54) **CANNULATION DEVICE**
PUNKTIONSVORRICHTUNG
DISPOSITIF DE CANULATION

(30) Priority: 10.06.2016 WO PCT/EP2016/173989
(43) Date of publication of application: 17.04.2019
(73) Proprietor: Fresenius Kabi Deutschland GmbH, 61352 Bad Homburg (DE)
(72) Inventor: AMON, Barbara, 65510 Idstein (DE)
(74) Representative: Fresenius Kabi Deutschland GmbH
(86) International application number: PCT/EP2017/059503
(87) International publication number: WO 2017/211493

(56) References cited:
- EP-A1- 0 653 220
- EP-A1- 1 683 542
- WO-A1-92/13584
- WO-A2-99/44655
- WO-A2-2006/070358
- GB-A- 2 503 329
- US-A- 5 129 884
- US-A1- 2007 191 772

## Description

The invention relates to a cannulation device for providing an access to a patient through a skin of the patient according to claim 1.

For placing a catheter in a human hollow organ, such as a visceral cavity (for example the gastro intestinal tract) through a patient's skin a cannulation device is used that is adapted to provide an access to the hollow organ through the patient's skin. The cannulation device comprises a puncture needle with a needle tip for puncturing the skin of the patient and a cannula for providing an access to the hollow organ through the puncture site.

From the state of the art a cannulation device with a puncture needle and a cannula is known wherein the tip of the puncture needle protrudes out of the cannula. The protruding needle tip may be a safety risk for the user as the user may hurt himself with the protruding tip by accident. Before puncturing the patient's skin the user may just cause a cutting injury with the needle tip. After puncturing the patient's skin and removing the cannulation device out of the patient's body, bacteria or viruses may be transmitted from the patient to the user additionally to the puncturing injury.

It is an object of the present invention to provide a cannulation device for providing an access to a patient through a skin of the patient that overcomes this safety risk.

To solve this problem the cannulation device of claim 1 is provided. This cannulation device comprises a puncture needle with a needle tip for puncturing the skin of the patient and a cannula. The puncture needle is movably connected to the cannula.

This allows a user to move the puncture needle with respect to the cannula such as to hide or cover the needle tip when desired, for instance before puncturing the patient's skin and after the patient's skin has been punctured and the cannulation device has been removed from the patient's body.

According to the invention, the puncture needle is arranged at least partially inside the cannula and the puncture needle is movable inside the cannula along a longitudinal axis of the cannulation device. The puncture needle and the cannula extend along the longitudinal axis of the cannulation device. The movable connection between the puncture needle and the cannula thus allows the puncture needle to slide within the cannula. For instance, the puncture needle is movable between a puncture position, wherein the needle tip protrudes out of the cannula, and a safety position, wherein the needle tip is arranged inside the cannula. In the puncture position the puncture needle is adapted to puncture a patient's skin, while in the safety position the tip of the puncture needle is covered by the cannula avoiding that a user hurts himself with the needle tip. As long as the cannulation device is introduced in the patient the cannula provides an access to the hollow organ through the puncture site. Outside the patient the cannula serves as a protective cover of the needle tip. The cannula thus has a double function. The needle tip may be moved out of the safety position into the puncture position only during the puncture procedure.

According to the invention, the puncture needle is secured to a needle holder and the cannula is secured to a cannula holder. The puncture needle and the cannula both have a distal end that is provided to be introduced into the patient and a proximal end opposite the distal end. The puncture needle and the cannula are secured to the needle holder and the cannula holder, respectively, at their proximal ends. For instance the needle holder and the cannula holder have a common extension range along the longitudinal axis of the cannulation device. The needle holder and the cannula holder extend concentrically about the longitudinal axis of the cannulation device. The needle holder is movably, for instance slidably, mounted at the cannula holder. For this purpose the cannula holder (needle holder) may comprise a guiding channel for receiving a portion of the needle holder (cannula holder) that may be guided within this guiding channel along the longitudinal axis of the cannulation device with respect to the cannula holder (needle holder).

According to the invention, the needle holder and the cannula holder each comprise at least one connecting element. The at least one connecting element of the needle holder and the at least one connecting element of the cannula holder are adapted to cooperate with each other such as to secure (lock) the needle holder with respect to the cannula holder in one or more defined position(s). The at least one connecting element of the needle holder and the at least one connecting element of the cannula holder may establish a detachable connection, for instance a latching connection. The at least one connecting element of the needle holder and the at least one connecting element of the cannula holder are adapted to cooperate with each other such as to hold (lock) the puncture needle in the safety position. This ensures that the puncture needle and in particular the needle tip does not accidentally moves out of the cannula. Additionally the at least one connecting element of the needle holder and the at least one connecting element of the cannula holder may be adapted to cooperate with each other such as to hold (lock) the puncture needle in the puncture position. This may help the user to hold the puncture needle (in particular the needle tip) in a defined (locked) position with respect to the cannula during the puncture procedure facilitating the manipulation of the cannulation device during the puncture procedure. Alternatively, in the puncture position the puncture needle may not be locked with respect to the cannula.

For instance, one connecting element may be provided for the needle holder (cannula holder) and two connecting elements may be provided for the cannula holder (needle holder). The two connecting elements of the cannula holder (needle holder) may be arranged in a distance to each other on an axis that extends substantially along the longitudinal axis of the cannulation device. The connecting element of the cannula holder that is arranged further away from the needle tip may be in engagement with the connecting element of the needle holder, when the puncture needle is in the safety position, while the connecting element of the cannula holder that is arranged closer to the needle tip may be in engagement with the connecting element of the needle holder, when the puncture needle is in the puncture position. Accordingly, in case that the needle holder comprises two connecting elements and the cannula holder one connecting element, the connecting element of the needle holder that is arranged further away from (closer to) the needle tip may be in engagement with the connecting element of the cannula holder, when the puncture needle is in the puncture position (safety position). By way of example, the one of the needle holder and the cannula holder has been described with one connecting element and the other of the needle holder and the cannula holder has been described with two connecting elements in order to lock the puncture needle with respect to the cannula in two different positions (safety position and puncture position). However, the number of connecting elements of the other of the needle holder and the cannula holder may be different from (greater than) two, if a different (greater) number of locking positions needs to be provided. Also, the number of connecting elements of the one of the needle holder and the cannula holder may be different from one, for instance in order to enhance the force of cooperation between the connecting elements.

Alternatively, one connecting element may be provided for the needle holder and one connecting element may be provided for the cannula holder. The connecting elements may be in engagement with each other when the puncture needle is in the safety position.

The at least one connecting element of the needle holder (cannula holder) may be a protrusion and the at least one connecting element of the cannula holder (needle holder) may be a recess or opening adapted to receive the protrusion such as to lock the protrusion.

According to an aspect, the puncture needle is moved from the safety position towards/into the puncture position by applying a force on the needle holder. The force may be applied linearly, for instance axially along the longitudinal axis of the cannulation device (in a direction from the proximal end to the distal end of the puncture needle). This allows using the cannulation device with a single hand: while the cannulation device is hold in one hand (at its cannula holder and/or needle holder) the force may be applied linearly with a thumb of this hand. As an alternative, the force may be applied along a helical path for instance. The force applied on the needle holder may also serve to bring the at least one connecting element of the needle holder and the at least one connecting element of the cannula holder out of engagement. As the puncture needle is secured to the needle holder, the force applied to the needle holder directly correlates with the speed of the puncture needle and the distance over which the puncture needle is moved. This allows a user to control precisely the puncture needle avoiding injuries by applying a force on the needle holder in order to move the puncture needle from the safety position towards/into the puncture position.

According to a further aspect, a release device may be provided that is adapted to release the at least one connecting element of the needle holder (cannula holder) out of engagement with the at least one connecting element of the cannula holder (needle holder). This release device may be provided to disengage the connecting elements holding the puncture needle in the puncture position. After disengagement the puncture needle may be moved out of the puncture position into/towards the safety position. A further release device may be provided to disengage the connecting elements holding the puncture needle and the needle tip in the safety position. After disengagement the puncture needle may be moved out of the safety position into/towards the puncture position.

An elastic element may be provided that is adapted to move the puncture needle and the needle tip out of the puncture position into the safety position. For this purpose the elastic element may be deformable along the longitudinal axis of the cannulation device. For example, the elastic element may be a spring element, such as a compressible/extendable (plastic) element or a (metallic) coil spring that extends along the longitudinal axis of the cannulation device. The elastic element may be out of its equilibrium configuration (for example stretched or compressed) when the puncture needle is in the puncture position and in or closer to its equilibrium configuration when the puncture needle is in the safety position. The restoring force of the elastic element may be used to drive the puncture needle from the puncture position into/towards the safety position. The puncture needle and the needle tip thus reproducibly move from the puncture position into/towards the safety position without requiring any contribution by the user. The user may however control the release device for disengaging the connecting elements holding the puncture needle in the puncture position, thus allowing the elastic element to move (after disengagement) the puncture needle and the needle tip out of the puncture position into/towards the safety position. The elastic element may be arranged such with respect to the needle holder and the cannula holder that it is brought out of its equilibrium configuration by the movement of the needle holder with respect to the cannula holder when the puncture needle is moved into/towards the puncture position.

As an alternative the elastic element may be adapted to move the puncture needle out of the safety position into the puncture position. In this case, the elastic element is out of its equilibrium configuration (for example stretched or compressed) when the puncture needle is in the safety position and in or closer to its equilibrium configuration when the puncture needle is in the puncture position. The restoring force of the elastic element is thus used to drive the puncture needle from the safety position into/towards the puncture position. All aspects considered for the case where the elastic element is adapted to move the puncture needle out of the puncture position into the safety position apply vice versa to the present case where the elastic element is adapted to move the puncture needle out of the safety position into the puncture position.

According to a further aspect, the needle holder comprises a hollow channel that extends through the entire needle holder along the longitudinal axis of the cannulation device. The puncture needle may be arranged (with its proximal end) at least in a part of the hollow channel. The hollow channel may have at least partially an inner diameter that corresponds to the outer diameter of the puncture needle, such that the puncture needle is hold in the hollow channel by frictional force. Additionally, the puncture needle may be glued into the hollow channel

The needle holder may further comprise a valve that is arranged in the hollow channel. The valve may be arranged in a distance to the proximal end of the puncture needle. The valve may be adapted to provide and block a fluid connection between the distal end of the puncture needle (that is intended to be inserted into the human body) on the one hand and the proximal end of the needle holder (that is intended to stay outside the human body) on the other hand.

According to a further aspect, the needle holder and the cannula holder form an assembly that is adapted to be gripped and hold by a user's hand allowing a user to manipulate the cannulation device with a single hand, for example.

The idea underlying the invention is shown in the figures. The parts in the figures are not necessarily to scale, instead emphasis being placed upon illustrating principles of the invention. Moreover, in the figures, like reference numerals designate corresponding parts. In the drawings:
- Fig. 1: schematically shows a cross-sectional view of a cannulation device according to an embodiment with a puncture needle and a cannula, wherein the puncture needle is in a safety position;
- Fig. 2: schematically shows a perspective view of the cannulation device of figure 1, wherein the puncture needle is in the safety position; and
- Fig. 3: schematically shows a perspective view of the cannulation device of figure 1, wherein the puncture needle is in a puncture position.

In the following detailed description, reference is made to the accompanying drawings which form a part hereof and in which are shown by way of illustration specific embodiments in which the invention may be practiced.

In this regard, it is to be understood that other embodiments may be utilized and structural or logical changes may be made without departing from the scope of the present invention. The following detailed description, therefore, is not to be taken in a limiting sense, and the scope of the present invention is defined by the appended claims.

Reference will now be made in detail to various embodiments, one or more examples of which are illustrated in the figures. Each example is provided by way of explanation, and is not meant as a limitation of the invention. For example, features illustrated or described as part of one embodiment can be used on or in conjunction with other embodiments to yield yet a further embodiment. It is intended that the present invention includes such modifications and variations. The examples are described using specific language which should not be construed as limiting the scope of the appended claims. The drawings are not scaled and are for illustrative purposes only. For clarity, the same elements have been designated by the same references in the different drawings if not stated otherwise.

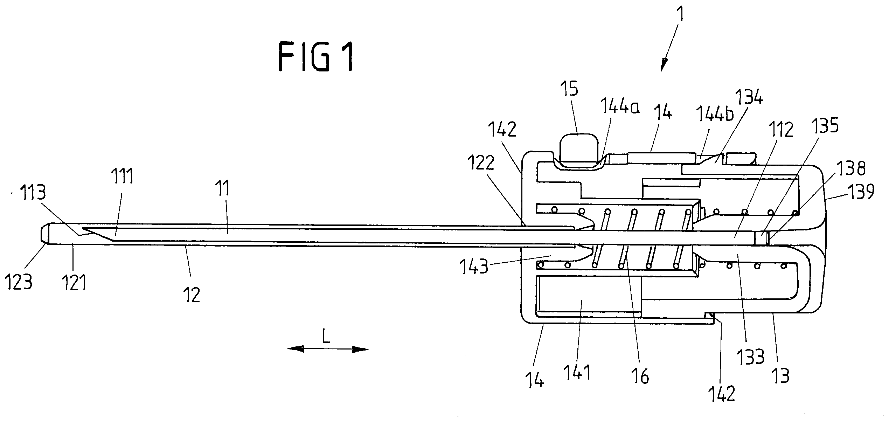

Figure 1 shows a sectional view of a cannulation device 1 according to one embodiment. The cannulation device 1 comprises a puncture needle 11 for puncturing the skin of a patient and a cannula 12 for providing an access to a hollow organ of the patient through the puncture site once the skin of the patient has been punctured by the puncture needle 11. The puncture needle 11 and the cannula 12 are both hollow tubes that extend along a longitudinal axis L of the cannulation device 1. The puncture needle 11 is arranged at least partially inside the cannula 12.

The puncture needle 11 and the cannula 12 both have a distal end 111 (with a needle tip), 121 (with a tapered tip) and a proximal end 112, 122 opposite to the distal end 111, 121 (seen along the longitudinal axis L of the cannulation device 1). The distal ends 111, 121 are provided to be introduced into the patient. At the distal end 111 of the puncture needle 11 a needle tip 113 is provided. The needle tip 113 is sharp-edged, and in particular bevelled, in order to puncture the patient's skin. At the distal end 121 of the cannula 12 a tapered tip 123 is provided. The tapered shape of the cannula 12 at its distal end allows for a smooth transition between the puncture needle 11 and the cannula 12.

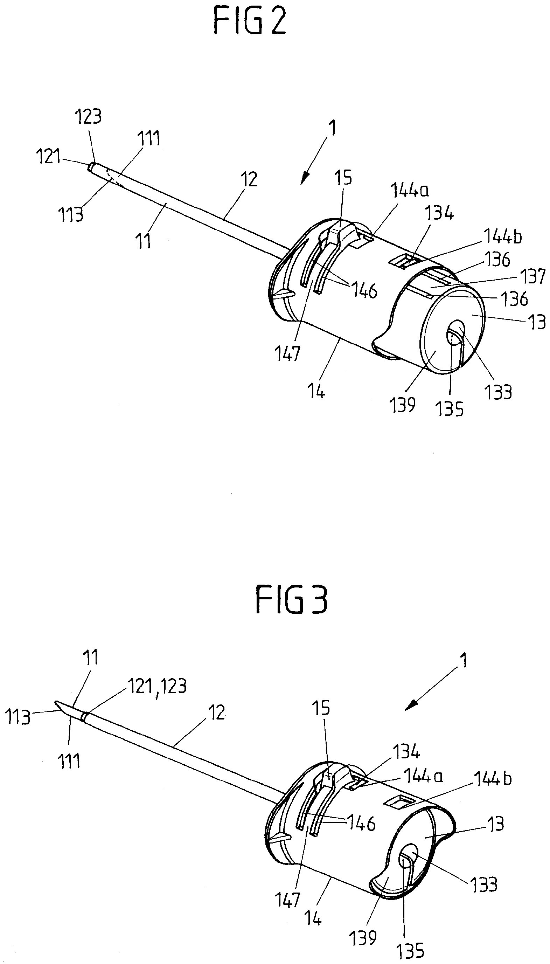

The puncture needle 11 is slidably arranged inside the cannula 12. The puncture needle 11 is movable with respect to the cannula 12 between a safety position (Figures 1 and 2) in which the needle tip 113 is arranged inside the cannula 12 and a puncture position (Figure 3) in which the needle tip 113 protrudes out of the cannula 12.

The puncture needle 11 is secured (at its proximal end 112) to a needle holder 13 while the cannula 12 is secured (at its proximal end 122) to a cannula holder 14. The puncture needle 11 and the cannula 12 are immovably attached to the needle holder 13 and the cannula holder 14, respectively. The needle holder 13 and the cannula holder 14 are dimensioned such that the cannulation device 1 can be hold and manipulated by a user at the needle holder 13 / cannula holder 14. As the needle holder 13 is immobile with respect to the puncture needle 11 and the cannula holder 14 is immobile with respect to the cannula 12, while the puncture needle 11 is slidable with respect to the cannula 12, also the needle holder 13 is slidable with respect to the cannula holder 14. The puncture needle 11 can thus be moved between the puncture position and the safety position by manipulating the needle holder 13 and/or cannula holder 14.

The cannula holder 14 is formed by a hollow body of substantially cylindrical shape that comprises a guiding channel 141 for receiving a portion of the needle holder 13 that is guided within the guiding channel 141 along the longitudinal axis L of the cannulation device 1 with respect to the cannula holder 14. The guiding channel 141 has a substantially annular cross section (in a plane perpendicular to the longitudinal axis L of the cannulation device 1) and is limited by two substantially cylindrical side walls (an inner side wall and an outer side wall). At its distal and proximal ends (oriented towards and away from the distal end 121 of the cannula 12) the cannula holder 142 comprises a stop element 142 that protrudes substantially perpendicularly to the longitudinal axis L of the cannulation device 1 into the guiding channel 141 such as to limit the path of the needle holder 13 inside the cannula holder 14. The distance along the longitudinal axis L may define the length of the path. At its proximal end the cannula holder 14 is open such that the guiding channel 141 may receive a portion of the needle holder 13. At its distal end the cannula holder 14 further comprises a sleeve 143 for holding the proximal end 122 of the cannula 12. The sleeve 143 extends substantially centrally inside the cylindrical side walls of the cannula holder 14 along the longitudinal axis L. The sleeve 143 has an inner diameter that corresponds to the outer diameter of the cannula 12, such that the cannula 12 may be hold by the sleeve 143 by frictional forces.

The needle holder 13 is also formed by a hollow body of substantially cylindrical shape. The substantially cylindrical side wall of the needle holder is dimensioned such that it is adapted to slide within the guiding channel 141 of the cannula holder 14 along the longitudinal axis L of the cannulation device 1. At its proximal end the needle holder 13 comprises a sleeve 133 for holding the proximal end 112 of the puncture needle 11. The sleeve 133 extends substantially centrally inside the cylindrical side wall of the needle holder 13 along the longitudinal axis L. The sleeve 133 is connected to the cylindrical side wall of the needle holder 13 by a push-surface 139 that extends substantially perpendicular to the longitudinal axis L. The push-surface 139 may be used to manually push the needle holder 13 into the cannula holder 14. The push-surface 139 has the shape of a disk that extends over the entire gap between the cylindrical side wall of the needle holder 13 and the sleeve 133. Alternatively, the disk may also extend only over a portion of that gap.

The sleeves 133 and 143 are arranged coaxially with respect to each other such that the puncture needle 11 extends inside the cannula 12. The sleeves 133, 143 hold the puncture needle 11 and the cannula 12, respectively, by frictional forces.

In order to lock the puncture needle 11 in the safety position and in the puncture position, the needle holder 13 and the cannula holder 14 comprise connecting elements 134, 144a and 144b. By way of example the needle holder 13 comprises one connecting element 134 arranged on its side wall and the cannula holder 14 comprises two connecting elements 144a, 144b arranged on its outer side wall. The connecting elements 144a, 144b of the cannula holder 14 are arranged in a distance to each other along an axis that extends substantially parallel to the longitudinal axis L. On the one hand, the connecting element 134 of the needle holder 13 is adapted to cooperate with the connecting element 144a of the cannula holder 14 (that is closer to the distal end 121 of the cannula 12 than the connecting element 144b) in order to hold the puncture needle 11 in the puncture position. On the other hand, the connecting element 134 of the needle holder 13 is adapted to cooperate with the connecting element 144b of the cannula holder 14 (that is further away from the distal end 121 of the cannula 12 than the connecting element 144a) in order to hold the puncture needle 11 in the safety position. In the embodiment shown in Figure 1, the connecting element 134 of the needle holder 13 is a protrusion that protrudes from the side wall of the needle holder 13 and extends in an angle to the longitudinal axis L towards the outer side wall of the cannula holder 14. The two connecting elements 144a, 144b of the cannula holder 14 are openings in the outer side wall of the cannula holder 14 that are adapted to receive the protrusion 134.

The shape (in particular the ratio of material thickness to the length along the longitudinal axis L) of the side wall of the needle holder 13 is chosen such to provide the side wall sufficient elasticity to bend when the protrusion 134 is entering or leaving one of the openings 144a, 144b or when the protrusion 134 is moving/located between the openings 144a, 144b. In order to enhance the elasticity, the side wall may comprise two grooves 136 that extend along the longitudinal axis L on both sides of the protrusion 134 (Figure 2). The grooves 136 define an oblong section 137 that extends along the longitudinal axis L and that supports the protrusion 134. The oblong shape of the section 137 allows this section to elastically bend with respect to the residual side wall of the needle holder 13 when the protrusion 134 is entering or leaving one of the openings 144a, 144b or when the protrusion 134 is moving/located between the openings 144a, 144b. The grooves 136 extend through the entire thickness of the side wall. As an alternative, the grooves 136 may be formed by reducing the material thickness of the side wall. As a further alternative the side wall of the needle holder 13 may be less elastic, but the protrusion 134 itself may be made of a material that has an elasticity allowing the protrusion 134 to deform (compress) when the protrusion 134 is leaving one of the openings 144a, 144b or when the protrusion 134 is moving/located between the openings 144a, 144b and to deform (expand) when the protrusion 134 is entering one of the openings 144a, 144b.

The protrusion 134 is moved out of engagement with the opening 144b (safety position) by pushing the needle holder 13 (at the push-surface 139) along the longitudinal axis L in a direction from the proximal end 112 to the distal end 111 of the puncture needle 11. The shape of the protrusion 134 allows the protrusion to slide out of the opening 144b when the needle holder 13 is pushed as described. When pushing the needle holder 13 further, the protrusion 134 moves from the opening 144b to the opening 144a. The elasticity of the side wall of the needle holder 13 (of the oblong section 137) allows the side wall (the oblong section 137) to slightly bend inside as long as the protrusion 134 is moving from the opening 144b to the opening 144a. When pushing the needle holder 13 further, the elasticity of the side wall of the needle holder 13 (of the oblong section 137) pushes the protrusion outwardly into engagement with the opening 144a (puncture position).

In order to release the protrusion 134 out of engagement with the opening 144a (puncture position) a release device 15 is provided at the outer side wall of the cannula holder 14 such that it is accessible for a user. The release device 15 is spring-mounted to the cannula holder 14. In the embodiment shown in Figures 2 and 3 the release device 15 is a protrusion formed on the outside of the outer side wall of the cannula holder 14. The outer side wall of the cannula holder 14 comprises two grooves 146 that extend in an angle to the longitudinal axis L on both sides of the release device 15. The grooves 146 define an oblong section 147 that extends in an angle to the longitudinal axis L and that supports the release device 15. The oblong shape of the section 147 allows this section to elastically bend with respect to the residual outer side wall of the cannula holder 14 upon application of a force on the release device 15. The grooves 146 extend through the entire thickness of the outer side wall. As an alternative, the grooves 146 may be formed by reducing the material thickness of the outer side wall.

The elasticity of the connection between the release device 15 and the cannula holder 14 (o the oblong section 147) allows the release device 15 to move (upon application of a corresponding force by a user) substantially perpendicularly to the longitudinal axis L from its rest position towards the protrusion 134 in order to push the protrusion 134 out of the opening 144a. When the user ceases to apply the force to the release device 15 the release device returns into its rest position.

In order to move the puncture needle 11 from the puncture position into the safety position once the engagement of the protrusion 134 and the opening 144a has been released, an elastic element 16 is provided. In the embodiment of Figure 1 the elastic element 16 is a coil spring that extends along the longitudinal axis L. The elastic element 16 is arranged between the inner side wall of the cannula holder 14 and the sleeve 143 of the cannula holder 14. The elastic element 16 also extends around the sleeve 133 of the needle holder 13. When the puncture needle 11 is in the puncture position, the coil spring 16 is out of its equilibrium configuration (compressed). Once the engagement of the protrusion 134 and the opening 144a has been released, the compressed coil spring 16 urges the needle holder 13 (in a direction from the distal end 111 to the proximal end 112 of the puncture needle 11) towards the safety position. When the puncture needle 11 has reached its safety position, the coil spring 16 is in or closer to its equilibrium configuration. Once the puncture needle 11 has reached the safety position the protrusion 134 engages the opening 144b due to the elasticity of the side wall of the needle holder 13 (of the oblong section 137 of the needle holder 13).

The needle holder 13 comprises a hollow channel 135 that extends through the entire needle holder 13 along the longitudinal axis L and substantially centrally through the sleeve 133. The puncture needle 11 that is hold by the sleeve 133 is arranged in a part of the hollow channel 135. When the cannulation device 1 is introduced in a hollow organ of a patient through the patient's skin, the hollow channel 135 in combination with the puncture needle 11 forms a fluid communication between the hollow organ and the patient's environment. As the hollow organ needs to be insufflated for placing a catheter, gas that is present in the hollow organ may flow out of the hollow organ through this fluid communication when puncturing the patient. In order to avoid that the insufflation gas flows out of the hollow organ, a valve 138 is arranged in the hollow channel 135 in a part of the sleeve 133. The valve 138 may be designed such that it allows insufflation of the hollow organ through the hollow channel 135 and the puncture needle 11.

Figure 2 shows a perspective view of the cannulation device 1 of Figure 1 with the puncture needle 11 in the safety position and Figure 3 shows a perspective view of the cannulation device 1 of Figure 1 with the puncture needle 11 in the puncture position. Generally, the outer diameter of the puncture needle 11 and the inner diameter of the cannula 12 are substantially identical and constant over the entire length (along the longitudinal axis L) in order to avoid the formation of a channel between the puncture needle 11 and the cannula 12. As an alternative the outer diameter of the puncture needle 11 is constant over the entire length and the inner diameter of the cannula 12 changes over its length, wherein the inner diameter of the cannula 12 is in at least one section (for instance at the distal end 121 of the cannula 12) of the cannula 12 substantially identical to the outer diameter of the puncture needle11.

## Claims

1. Cannulation device (1) for providing an access to a patient through a skin of the patient, comprising a puncture needle (11) with a needle tip (113) for puncturing the skin of the patient and a cannula (12)
**characterized in**
**that** the puncture needle (11) is movably connected to the cannula (12),
wherein the puncture needle (11) is movable between a puncture position, wherein the needle tip (113) protrudes out of the cannula (12), and a safety position,
wherein the needle tip (113) is arranged inside the cannula (12),
wherein the puncture needle (11) is secured to a needle holder (13) and the cannula (12) is secured to a cannula holder (14), wherein the needle holder (13) is movably mounted at the cannula holder (14),
wherein the needle holder (13) and the cannula holder (14) each comprise at least one connecting element (134, 144a, 144b), wherein the at least one connecting element (134) of the needle holder (13) and the at least one connecting element (144a, 144b) of the cannula holder (14) are adapted to cooperate with each other such as to secure the needle holder (13) with respect to the cannula holder (14) and to hold the puncture needle (11) in the safety position.

2. Cannulation device (1) according to claim 1, **characterized in that** the puncture needle (11) is arranged at least partially inside the cannula (12) and wherein the puncture needle (11) is movable inside the cannula (12) along a longitudinal axis (L) of the cannulation device (1).

3. Cannulation device (1) according to claim 1 or 2, **characterized in that** the at least one connecting element (134) of the needle holder (13) is brought out of engagement with the at least one connecting element (144b) of the cannula holder (14) for moving the puncture needle (11) out of the safety position into the puncture position by applying a force on the needle holder (13).

4. Cannulation device (1) according to any of the preceding claims, **characterized by** a release device (15) that is adapted to release the at least one connecting element (134) of the needle holder (13) out of engagement with the at least one connecting element (144a) of the cannula holder (14) for moving the puncture needle (11) out of the puncture position into the safety position.

5. Cannulation device (1) according to any of the preceding claims, **characterized by** an elastic element (16) adapted to move the puncture needle (11) out of the puncture position into the safety position.

6. Cannulation device (1) according to claim 5, **characterized in that** the elastic element (16) is out of its equilibrium configuration when the puncture needle (11) is in the puncture position and in or closer to its equilibrium configuration when the puncture needle (11) is in the safety position.

7. Cannulation device (1) according to any of the preceding claims, **characterized in that** the needle holder (13) comprises a hollow channel (135) that extends through the entire needle holder (13) and **in that** the puncture needle (11) is arranged in a part of the hollow channel (135).

8. Cannulation device (1) according to claim 7, **characterized in that** the needle holder (13) comprises a valve (138) that is arranged in the hollow channel (135).

9. Cannulation device (1) according to any of the preceding claims, **characterized in that** the needle holder (13) and the cannula holder (14) form an assembly that is adapted to be gripped and hold by a user's hand.

## Patentansprüche

1. Kanüliervorrichtung (1) zum Bereitstellen eines Zugangs zu einem Patienten durch eine Haut des Patienten, umfassend eine Einstichnadel (11) mit einer Nadelspitze (113) zum Durchstechen der Haut des Patienten und eine Kanüle (12)
**dadurch gekennzeichnet, dass**
die Einstichnadel (11) beweglich mit der Kanüle (12) verbunden ist,
wobei die Einstichnadel (11) zwischen einer Einstichposition, wobei die Nadelspitze (113) aus der Kanüle (12) herausragt, und einer Sicherheitsposition, wobei die Nadelspitze (113) in der Kanüle (12) angeordnet ist, beweglich ist,
wobei die Einstichnadel (11) an einem Nadelhalter (13) gesichert ist und die Kanüle (12) an einem Kanülenhalter (14) gesichert ist, wobei der Nadelhalter (13) beweglich in dem Kanülenhalter (14) installiert ist,
wobei der Nadelhalter (13) und der Kanülenhalter (14) jeweils mindestens ein Verbindungselement (134, 144a, 144b) umfassen, wobei das mindestens eine Verbindungselement (134) des Nadelhalters (13) und das mindestens eine Verbindungselement (144a, 144b) des Kanülenhalters (14) ausgelegt sind, um zusammenzuwirken, um den Nadelhalter (13) in Bezug auf den Kanülenhalter (14) zu sichern und um die Einstichnadel (11) in der Sicherheitsposition zu halten.

2. Kanüliervorrichtung (1) nach Anspruch 1, **dadurch gekennzeichnet, dass** die Einstichnadel (11) mindestens teilweise innerhalb der Kanüle (12) angeordnet ist und wobei die Einstichnadel (11) innerhalb der Kanüle (12) entlang einer Längsachse (L) der Kanüliervorrichtung (1) beweglich ist.

3. Kanüliervorrichtung (1) nach Anspruch 1 oder 2, **dadurch gekennzeichnet, dass** das mindestens eine Verbindungselement (134) des Nadelhalters (13) außer Eingriff mit dem mindestens einen Verbindungselement (144b) des Kanülenhalters (14) zum Herausbewegen der Einstichnadel (11) aus der Sicherheitsposition in die Einstichposition durch Aufbringen einer Kraft auf den Nadelhalter (13) gebracht wird.

4. Kanüliervorrichtung (1) nach einem der vorhergehenden Ansprüche, **gekennzeichnet durch** eine Entriegelungsvorrichtung (15), die ausgelegt ist, um das mindestens eine Verbindungselement (134) des Nadelhalters (13) außer Eingriff mit dem mindestens einen Verbindungselement (144a) des Kanülenhalters (14) zu bringen, um die Einstichnadel (11) aus der Einstichposition in die Sicherheitsposition zu bewegen.

5. Kanüliervorrichtung (1) nach einem der vorhergehenden Ansprüche, **gekennzeichnet durch** ein elastisches Element (16), das geeignet ist, die Einstichnadel (11) aus der Einstichposition in die Sicherheitsposition zu bewegen.

6. Kanüliervorrichtung (1) nach Anspruch 5, **dadurch gekennzeichnet, dass** sich das elastische Element (16) außerhalb seiner Gleichgewichtskonfiguration befindet, wenn sich die Einstichnadel (11) in der Einstichposition befindet, und in oder näher an seiner Gleichgewichtskonfiguration, wenn sich die Einstichnadel (11) in der Sicherheitsposition befindet.

7. Kanüliervorrichtung (1) nach einem der vorhergehenden Ansprüche, **dadurch gekennzeichnet, dass** der Nadelhalter (13) einen Hohlkanal (135) aufweist, der sich durch den gesamten Nadelhalter (13) erstreckt, und dadurch, dass die Einstichnadel (11) in einem Teil des Hohlkanals (135) angeordnet ist.

8. Kanüliervorrichtung (1) nach Anspruch 7, **dadurch gekennzeichnet, dass** der Nadelhalter (13) ein Ventil (138) umfasst, das in dem Hohlkanal (135) angeordnet ist.

9. Kanüliervorrichtung (1) nach einem der vorhergehenden Ansprüche, **dadurch gekennzeichnet, dass** der Nadelhalter (13) und der Kanülenhalter (14) eine Anordnung bilden, die ausgelegt ist, um von einer Benutzerhand ergriffen und gehalten zu werden.

## Revendications

1. Dispositif de canulation (1) pour fournir un accès à un patient à travers une peau du patient, comprenant une aiguille de ponction (11) avec une pointe d'aiguille (113) pour percer la peau du patient et une canule (12)
**caractérisé en**
**ce que** l'aiguille de ponction (11) est raccordée de manière mobile à la canule (12),
dans lequel l'aiguille de ponction (11) est mobile entre une position de ponction, dans laquelle la pointe d'aiguille (113) fait saillie hors de la canule (12), et une position de sécurité, dans laquelle la pointe d'aiguille (113) est disposée à l'intérieur de la canule (12),
dans lequel l'aiguille de ponction (11) est fixée à un porte-aiguille (13) et la canule (12) est fixée à un porte-canule (14), dans lequel le porte-aiguille (13) est monté de manière mobile sur le porte-canule (14),
dans lequel le porte-aiguille (13) et le porte-canule (14) comprennent chacun au moins un élément de raccordement (134, 144a, 144B), dans lequel l'au moins un élément de raccordement (134) du porte-aiguille (13) et l'au moins un élément de raccordement (144a, 144b) du porte-canule (14) sont adaptés pour coopérer les uns avec les autres de manière à fixer le porte-aiguille (13) par rapport au porte-canule (14) et pour maintenir l'aiguille de ponction (11) en position de sécurité.

2. Dispositif de canulation (1) selon la revendication 1, **caractérisé en ce que** l'aiguille de ponction (11) est disposée au moins partiellement à l'intérieur de la canule (12) et dans lequel l'aiguille de ponction (11) est mobile à l'intérieur de la canule (12) le long d'un axe longitudinal (L) du dispositif de canulation (1).

3. Dispositif de canulation (1) selon la revendication 1 ou 2, **caractérisé en ce que** l'au moins un élément de raccordement (134) du porte-aiguille (13) est amené hors de prise avec l'au moins un élément de raccordement (144b) du porte-canule (14) pour déplacer l'aiguille de ponction (11) hors de la position de sécurité dans la position de ponction en appliquant une force sur le porte-aiguille (13).

4. Dispositif de canulation (1) selon l'une quelconque des revendications précédentes, **caractérisé par** un dispositif de libération (15) qui est est adapté pour libérer l'au moins un élément de raccordement (134) du porte-aiguille (13) hors de prise avec l'au moins un élément de raccordement (144a) du porte-canule (14) pour déplacer l'aiguille de ponction (11) hors de la position de ponction dans la position de sécurité.

5. Dispositif de canulation (1) selon l'une quelconque des revendications précédentes, **caractérisé par** un élément élastique (16) adapté pour déplacer l'aiguille de ponction (11) hors de la position de ponction dans la position de sécurité.

6. Dispositif de canulation (1) selon la revendication 5, **caractérisé en ce que** l'élément élastique (16) est hors de sa configuration d'équilibre lorsque l'aiguille de ponction (11) est dans la position de ponction et dans ou plus proche de sa configuration d'équilibre lorsque l'aiguille de ponction (11) est dans la position de sécurité.

7. Dispositif de canulation (1) selon l'une quelconque des revendications précédentes, **caractérisé en ce que** le porte-aiguille (13) comprend un canal creux (135) qui s'étend à travers tout le porte-aiguille (13) et **en ce que** l'aiguille de ponction (11) est disposée dans une partie du canal creux (135).

8. Dispositif de canulation (1) selon la revendication 7, **caractérisé en ce que** le porte-aiguille (13) comprend une valve (138) qui est disposée dans le canal creux (135).

9. Dispositif de canulation (1) selon l'une quelconque des revendications précédentes, **caractérisé en ce que** le porte-aiguille (13) et le porte-canule (14) forment un ensemble qui est adapté pour être saisi et tenu par la main d'un utilisateur.
